# EUROPEAN PATENT APPLICATION

(11) **EP 1 982 971 A1**
(43) Date of publication of application: **22.10.2008**
(21) Application number: 06843216.0
(22) Date of filing: 26.12.2006
(51) Int. Cl.: C07C 37/84, B01D 9/02, C07C 37/20, C07C 39/16, C07B 61/00

(54) **PROCESS AND EQUIPMENT FOR THE RECOVERY OF BISPHENOL A**

(30) Priority: 02.02.2006 JP 2006025720
(71) Applicant: Idemitsu Kosan Co., Ltd., Chiyoda-ku Tokyo 100-8321 (JP); Tsukishima Kikai Co., Ltd., Tokyo 104-0051 (JP)
(72) Inventor: YOSHITOMI, Kazuyuki, Chiba 299-0193 (JP); KODAMA, Masahiro, Chiba 299-0193 (JP); MASUDA, Shuichi, Chiba 299-0193 (JP); TAKEGAMI, Keizou, Tokyo 104-0051 (JP); SUDA, Hideki, Tokyo 104-0051 (JP)
(74) Representative: Vossius & Partner
(86) International application number: PCT/JP2006/325832
(87) International publication number: WO 2007/088689

(57) **Abstract**

In a process for recovering bisphenol A from an isomerized liquid, a method for recovering bisphenol A is **characterized** as: a liquid isomerized in step (D) is supplied to a crystallizer having an external jacket and a function of scratching off a deposit on the inside wall of the crystallizer with a scraper blade while cooling water is passed through the external jacket, so that the inside of the crystallizer is cooled to crystallize an adduct of bisphenol A and phenol in the presence of phenol; the adduct deposited on the inside wall of the crystallizer is scratched off; a slurry liquid containing the adduct scratched off is filtered and washed with a batch-type filter that has a washing function and serves as a solid/liquid separator to recover a bisphenol A adduct; and the recovered bisphenol A adduct is returned to a concentration step and/or a crystallization and solid/liquid separation step. An apparatus for recovering bisphenol A includes a crystallizer having a jacket and a function of scratching off with a scraper blade and a batch-type filter having a washing function.

## Description

### [Technical Field]

The present invention relates to a method and an apparatus for recovering bisphenol A from an isomerized liquid that is treated in an isomerization step, highly viscous, and contains a large amount of impurities. More specifically, the present invention relates to a method for recovering bisphenol A, that is characterized as: in a recovering step, with a crystallizer that is equipped with a jacket and has a function of scratching off with a scraper blade an adduct of bisphenol A and phenol (hereinafter, referred to as bisphenol A adduct simply) deposited on the inside wall of the crystallizer by cooling it with cooling water flowing through an external jacket, the bisphenol A adduct is crystallized; a slurry containing the bisphenol A adduct obtained is filtered and washed with a batch-type filter that has a washing function and serves as a solid/liquid separator; the resulting bisphenol A adduct is returned to a concentration step and/or a crystallization and solid/liquid separation step, and the present invention also relates to an apparatus for recovering bisphenol A, which includes the crystallizer equipped with the jacket and the batch-type filter having a washing function.

### [Background Art]

A method for producing bisphenol A used as a source material for polycarbonate, which is an engineering plastics having growing demand in recent years, usually includes a recovering step. In the recovering step, a part of a liquid containing impurities produced in a condensation reaction step in which an excess amount of phenol is reacted with acetone in the presence of an acidic catalyst is discharged out of the reaction system so as not to concentrate the impurities in the production apparatuses. After valuable components are recovered from the discharged liquid, the resulting residue is disposed as tar.

As the above method for recovering, for example, Patent Document 1 discloses a method for recovering valuable components from the discharged liquid using a process named as the alkaline decomposition method. In this method, a little amount of an alkaline substance such as NaOH is added, then bisphenol A and impurities that are contained in the liquid are recovered as a light fraction such as phenol and isopropenylphenol by using a reactive still at a high temperature above 200°C under a reduced pressure, while a heavy fraction is discharged as tar. This method has a disadvantage of a high installation cost due to high temperature treatment in the presence of the alkaline substance. In addition, alkali is admixed in the tar, so that the tar is not easy to dispose, thereby this method has another disadvantage of a high installation cost for tar disposal.

In another method besides the alkaline decomposition method, the bisphenol A adduct contained in the discharged liquid is crystallized by using the crystallization method; the adduct is subjected to solid/liquid separation; then bisphenol A is recovered from the bisphenol A adduct obtained after the solid/liquid separation.
The crystallization method includes an external cooling crystallization as described in Patent Document 1 in which the discharged liquid is cooled with a heat-exchanger so as to crystallize the bisphenol A adduct out of the discharged liquid, and a vacuum-vaporization crystallization as described in Patent Document 2 in which the discharge liquid and water are introduced into a crystallizer so as to perform crystallization by cooling with the latent heat of water vaporization under a reduced pressure.
However, either crystallization method has such a disadvantage that a mother liquid is highly viscous. Further, in the case of the external cooling crystallization, a large amount of slurry liquid containing bisphenol A adduct crystals is required to be circulated through the heat-exchanger. The bisphenol A adduct crystals are fine needle crystals with an average particle size of 100 microns (minor axis) when phenol is used as a solvent. Thereby the following problem arises: the needle crystals are crushed by a blade of a circulation pump for cooling, so that liquid has poor filtrating property in the solid/liquid separation performed in the downstream and that the purity of the recovered crystals becomes lowered. In addition, crystals are deposited on the tube of the heat-exchanger, so that the scales are required to be dissolved out by elevating the temperature of the heat-exchanger, once per several months. During this period of dissolving out the scales, the discharged liquid is not supplied to the recovering process, thereby arises a problem of stopping the apparatus operation.
On the other hand, in the case of the vacuum-vaporization crystallization in which water is added to the mother liquid, there is no fear about crushing of crystals or stopping of apparatus operation, however, the solubility of bisphenol A in the mother liquid is remarkably increased as compared with a phenol solution because water is remained in the mother liquid in an amount of 3 to 5% by mass (for example, the solubility of bisphenol A in a phenol solvent containing 5% by mass of water is 14% by mass at 50°C as opposed to the solubility of bisphenol A in a phenol solvent is 9% by mass). Due to this, the solubility of bisphenol A increases, thereby arising a problem of lowering the percent of bisphenol A recovered in the form of crystals and becoming worse unit of raw material.

The solid/liquid separator in the recovery process is required to meet such a hard condition of recovering high-purity crystals from slurry that contains fine crystals by solid/liquid separation in the presence of highly viscous liquid.
The solid/liquid separator includes a centrifugal separator and a drum filter in general. When a centrifugal separator is applied as the solid/liquid separator in the recovery process, the recovery of the crystals becomes worse because fine crystals leak to the mother liquid through a screen of the centrifugal separator. In addition, due to high viscosity of the mother liquid and fine particle size of the crystals, the crystals are not easily washed. This causes another problem of making worse the parity of the recovered crystals.
Further, when a drum filter having a filter cloth provided on a rotation body is used, can be avoided a problem of leaking the crystals to the mother liquid through the filter cloth due to the fine mesh of the cloth, however, due to high viscosity of the mother liquid and fine particle size of the crystals, the staying time at the filter becomes short in the case of the drum filter. There arises a problem of becoming worse the purity of the recovered crystals due to insufficient filtration and lack of washing time.

Patent Document 1: Japanese Patent Laid-Open Publication No. 5-345737,
Patent Document 2: Japanese Patent Laid-Open Publication No. 2004-359594.

### [Disclosure of the Invention]

### [Problems to be Solved by the Invention]

The present invention has been made to address the problems set forth under the above circumstances. It is an object of the present invention to provide a method and an apparatus for recovering bisphenol A in a step of recovering bisphenol A from an isomerized liquid, that is one of the steps of a bisphenol A production process, in which a high-purity bisphenol A adduct is recovered efficiently and the recovered bisphenol A adduct is returned to a concentration step and/or a crystallization and solid/liquid separation step.

### [Means for Solving the Problems]

The present inventors have made intensive studies to achieve the above objective. As a result, the present inventors have found that the above objective can be achieved by: in a step of recovering bisphenol A from an isomerized liquid, with a crystallizer that is equipped with a jacket and has a function of scratching off with a scraper blade a bisphenol A adduct deposited on the inside wall of the crystallizer by cooling it with cooling water flowing through an external jacket and a batch-type filter that serves as a liquid-solid separator and has a washing unction, recovering the bisphenol A adduct by filtering and washing; and returning the recovered bisphenol A adduct to a concentration step and/or a crystallization and solid/liquid separation step. The present invention has been accomplished based on this finding.

Namely, the present invention provides:
(1) In a bisphenol A production process including the steps: (A) a condensation reaction step in which an excess amount of phenol is reacted with acetone in the presence of an acidic catalyst; (B) a concentration step in which a reaction mixture obtained in the step (A) is concentrated; (C) a crystallization and liquid-solid separation step in which the reaction mixture concentrated in the step (B) is cooled so as to crystallize an adduct of bisphenol A and phenol and to separate it into the adduct and a mother liquid; (D) an isomerization step in which the all of the mother liquid obtained in the step (C) is treated with an isomerization catalyst and isomerized; (E) a recovering step in which the adduct of bisphenol A and phenol is recovered from the isomerized liquid treated in the step (D); (F) an adduct decomposition step in which phenol is removed from the adduct of bisphenol A and phenol obtained in the step (C) to obtain a melt of bisphenol A; and (G) a prilling step in which the melt of bisphenol A is prilled into product prill,
   a method for recovering bisphenol A in the step (E), wherein the isomerized liquid treated in the step (D) is introduced in a crystallizer that is equipped with an external jacket and has a function of scratching off a deposit on the inside wall thereof with a scraper blade while cooling water is passed through the external jacket so as to cool the inside of the crystallizer, whereby the adduct of bisphenol A and phenol is crystallized in the presence of phenol; a slurry containing the adduct that is obtained by scratching off the adduct deposited on the inside wall of the crystallizer is filtered and washed with a batch-type filter having a washing function and serving as a solid/liquid separator; and the resulting adduct is circulated to the step (B) and/or step (C).
(2) The method for recovering bisphenol A as described in (1), wherein a washing liquid used in the batch-type filter having a washing function is selected from phenol, phenol containing water, and phenol containing bisphenol A.
(3) An apparatus for recovering bisphenol A includes a crystallizer having a jacket and a function of scratching off an adduct of bisphenol A and phenol deposited on an inside wall thereof with a scraper blade and a batch-type filter having a washing function.

### [Best Mode for Carrying Out the Invention]

The method for recovering bisphenol A according to the present invention is **characterized in that** among the following steps (A) to (G), in the recovering step (E), an isomerized liquid treated in the step (D) is introduced into a crystallizer that is equipped with an external jacket and has a function of scratching off a deposit on the inside wall thereof with a scraper blade, and cooling water is passed through the external jacket so as to cool the inside of the crystallizer in the presence of phenol and to crystallize an adduct of bisphenol A and phenol; a slurry containing the adduct that is obtained by scratching off the adduct deposited on the inside wall of the crystallizer is filtered and washed with a batch-type filter that has a washing function and serves as a solid/liquid separator; and the resulting adduct is circulated to the step (B) and/or step (C). Further, the apparatus for recovering according to the present invention includes a crystallizer that is equipped with a jacket and has a function of scratching off an adduct of bisphenol A and phenol deposited on the inside wall thereof with a scraper blade and a batch-type filter having a washing function.

(A) A condensation reaction step in which an excess amount of phenol is reacted with acetone in the presence of an acidic catalyst;
(B) A concentration step in which a reaction mixture obtained in the reaction step is concentrated;
(C) A crystallization and liquid-solid separation step in which a concentrated liquid obtained in the concentration step is cooled so as to crystallize an adduct of bisphenol A and phenol and to separate it into the adduct and a mother liquid;
(D) An isomerization step in which all of the mother liquid is treated with an isomerization catalyst and isomerized;
(E) A recovering step in which bisphenol A is recovered from a part of the isomerized liquid treated in the isomerization step;
(F) An adduct decomposition step in which phenol is removed from the adduct of bisphenol A and phenol obtained in the crystallization and solid/liquid separation step to obtain a melt of bisphenol A; and
(G) A prilling step in which the melt of bisphenol A is prilled into product prill.

Hereinafter, each step is explained.

### (A) Condensation reaction step

Source phenol and acetone are reacted in a manner that phenol is in excess stoichiometrically. The molar ratio of phenol to acetone, phenol/acetone, is usually in the range of from 3 to 30 and preferably from 5 to 20. The reaction temperature is usually from 50 to 100°C, and the reaction pressure is usually from normal pressure to 1.5 MPa and preferably from normal pressure to 0.6 MPa. The catalyst includes usually a strongly acidic cation-exchange resin such as a sulfonic acid type. Further, may be included a catalyst that is given by neutralizing a part of the strongly acidic cation-exchange resin with an auxiliary catalyst such as mercapto alkylamine. For example, may be included a catalyst whose sulfonic acid group is neutralized by from 5 to 30 mol% with 2-mercaptoethylamine, 3-mercaptopropylamine, N,N-dimethyl-3-mercaptopropylamine, N,N-di-n-butyl-4-mercaptobutylamine, 2,2-dimethylthiazolidine, and the like.
Reaction between phenol and acetone is carried out in a fixed-bed flow process that is a continuous and flow system or in a suspension-bed batch-wise process. In the case of the fixed-bed flow process, the liquid hourly space velocity (LHSV) of a source liquid supplied to a reactor is from about 0.2 to 50 hr⁻¹. In the case of the suspension-bed batch-wise process, the amount of a resin catalyst is in the range of generally from 20 to 100% by mass with respect to the source liquid and the reaction time is from about 0.5 to 5 hours, although they depend on the reaction temperature and reaction pressure.

### (B) Concentration step

The reaction mixture from the condensation reaction step is usually concentrated in two-steps. In a first concentration step, unreacted acetone, reaction product water, and others are removed by vacuum distillation or the like. Vacuum distillation is carried out at a temperature of from about 30 to 180°C under a pressure of from about 13 to 67 kPa. Then, in a second concentration step, phenol is distilled out so as to control the bisphenol A concentration. The bisphenol A concentration is controlled to be preferably from 20 to 60% by mass. At a bisphenol A concentration of lower than 20% by mass, yield becomes lowered. On the other hand, at a bisphenol A concentration of higher than 60% by mass, solidification temperature is elevated and solidification occurs easily, thereby bringing about a problem of rendering transportation impossible. Therefore, usually in the first concentration step, the reaction mixture is concentrated in advance and adjusted at a concentration within the above range. The second concentration step is usually performed preferably under the conditions of from about 4 to 40 kPa of pressure and from about 70 to 140°C of temperature.

### (C) Crystallization and solid/liquid separation step

The concentrated liquid from the concentration step is usually cooled from about 70 to 140°C to about 35 to 60°C, so that an adduct (adduct crystal) of bisphenol A and phenol is crystallized and that the concentrated liquid is changed into a slurry liquid. The concentrated liquid is cooled by heat removal using vaporization latent heat of water that is added to an external heat-exchanger or the crystallizer. After that, the slurry liquid is subjected to solid/liquid separation. The composition of the mother liquid obtained in the crystallization and solid/liquid separation step is usually as: from 65 to 85 % by mass of phenol, from 10 to 20% by mass of bisphenol A, and from 5 to 15% by mass of by-products such as 2,4-isomer. The mother liquid contains a large amount of impurities such as 2,4-isomer. All of the mother liquid is treated in the following isomerization step so as to convert the 2,4-isomer into bisphenol A and to recover phenol and bisphenol A from the mother liquid.
The adduct crystals separated from the slurry liquid by the solid/liquid separation are fed to the adduct decomposition step in which phenol is removed to obtain high-purity bisphenol A.
A solid that contains mainly the adduct crystals and is deposited on the surface of a filter of the solid/liquid separator after filtration is washed with a washing liquid. The washing liquid may include phenol that is recovered by evaporation, source phenol, water, and a water-phenol mixed liquid, and also include a solution similar to a bisphenol A saturated phenol solution. The much the amount of the washing liquid used the better of course from the viewpoint of washing efficiency. However, the amount has an own upper limit considering the dissolution loss of the adduct crystals, and the circulation, recovery and reuse of the washing liquid. The amount considered to be most efficient is usually from 0.1 time to 10 times of the crystals on a mass basis. Note that, the adduct crystal may be re-dissolved after the crystallization and solid/liquid separation, and the crystallization and solid/liquid separation may be repeated. The impurities incorporated in the adduct crystals are decreased successively by repeating the crystallization and solid/liquid separation in multi-stages. As a washing liquid used in this occasion to wash a solution obtained by re-dissolution and a solid that is obtained by liquid-solid separation and contains mainly the adduct, may be used in each stage phenol that is recovered by evaporation, source phenol, water, and a water-phenol mixed liquid, and also a solution similar to a phenol with satuerated bisphenol A. The mother liquid obtained by re-crystallization and solid/liquid separation may be recycled to the former crystallization stage.

### (D) Isomerization step

All of the liquid phase (mother liquid) obtained in the crystallization and solid/liquid separation step is supplied to the following isomerization step in which the reacted by-products contained in the mother liquid are isomerized. A part of the resulting isomerized liquid is circulated to at least any one of steps including the condensation reaction step, concentration step, and crystallization and solid/liquid separation step.
Further, a part of the resulting isomerized liquid is drawn out to prevent impurity accumulation and is fed to the recovering step as a discharged liquid.
The isomerization is performed using usually a sulfonic acid type cation-exchange resin as a catalyst, at a reaction temperature of from about 50 to 100°C and a liquid hourly space velocity (LHSV) of from about 0.2 hr⁻¹ to 50 hr⁻¹ in the case of a solid bed flow process that is a continuous flow system.

### (E) Recovering step

The discharged liquid from the isomerization step contains bisphenol A in an amount of from about 15 to 20% by mass and by-products such as 2,4-isomer in an amount of from about 5 to 10% by mass.
After this discharged liquid is concentrated, it is cooled in the presence of phenol to crystallize the adduct (adduct crystal) of bisphenol A and phenol, and is subjected to solid/liquid separation. The resulting adduct crystals are melted, and then circulated to the concentration step and/or crystallization and solid/liquid separation step. The mother liquid remained after the solid/liquid separation is disposed after phenol is recovered from it.

Next, the recovering step is explained in more detail.
The discharged liquid from the isomerization step is concentrated by removing a part of phenol with an evaporator or the like. The evaporated phenol may be reused as a washing liquid for washing the bisphenol A adduct (hereinafter, referred to as adduct crystals) that is separated from liquid with a batch-type filter in the recovering step.
The bisphenol A concentration in the liquid after concentrated is from about 20 to 50% by mass. The concentration operation with the evaporator or the like is performed under a pressure of from about 5.3 to 40 kPa at a temperature of from about 70 to 140°C. Thus obtained concentrated liquid of the discharged liquid is cooled in the crystallizer in which the liquid is rendered to a slurry liquid by crystallizing the adduct crystals, and then separated into solid and liquid with the batch-type filter. The concentrated liquid may be supplied to the crystallizer after it is cooled to a temperature near the solidification point with a heat-exchanger using hot water as a cooling medium. These adduct crystals (hereinafter, also referred to as recovery adduct) obtained by solid/liquid separation with the batch-type filter is returned back to the aforementioned concentration step (second concentration step) or crystallization and solid/liquid separation step.
The crystallizer in which the recovery adduct is crystallized out of the concentrated liquid may include a crystallizer (for example, a scratch-type crystallizer, manufactured by Tsukishima Kikai Co., Ltd.) that is characterized by flowing cooling water through an external jacket and having a function of scratching off with a scraper blade the recovery adduct deposited on the inside wall of the crystallizer. The present crystallizer, in which the recovery adduct deposited on the cooled inside wall of the crystallizer is scratched off constantly so as to renew the inside wall and the slurry liquid of phenol containing the recovery adduct is circulated slowly inside of the crystallizer so as to grow the recovery adduct, has a capability of preventing unnecessary crushing of crystals.

The scratch-type crystallizer that has a capability of providing sufficient inside circulation does not cause a problem of sticking recovery adduct blocks to the inside of the crystallizer, so that the crystallizer is suitably used in the recovery step in which the impurity concentration and viscosity are high and a continuous stable operation is required over a long period of time.
The crystallization temperature is preferably from 35 to 60°C and more preferably from 45 to 50°C. When the crystallization temperature is lower than 35°C, both phenol and bisphenol A become solid. On the other hand, at over 60°C, the bisphenol A solubility in phenol abruptly increases, as a result, the recovery percentage of bisphenol A crystals is lowered.
The difference between the temperature of the cooling water flowing through the external jacket of the scratch-type crystallizer and the inside temperature of the crystallizer is desirably from about 10 to 20°C. The difference lower than 10°C makes small the cooling effect, so that the size of the crystallizer is required to be increased. This is economically disadvantageous. On the other hand, when the difference goes over 20°C, the adduct is deposited too thick on the inside cooled face of the crystallizer to be scratched off with a scraper blade.

The slurry liquid that is prepared by cooling and crystallizing the recovery adduct in the crystallizer is then filtered and washed with a batch-type filter. Examples of the batch-type filter may include a tray filter manufactured by Tsukishima Kikai Co., Ltd.
Because the slurry liquid from the crystallizer contains fine crystals (needle crystals) and the impurity concentration and viscosity of the mother liquid are high, the slurry liquid has an extremely poor filtrating property. In order to obtain high-purity crystals, filtration or washing operation within a time scale of minutes is required, so that a drum filter or a centrifugal separator that treats the slurry within a time scale of seconds is not appropriate as a solid/liquid separator in the recovery step. The batch-type filter is suitably satisfied this condition.
A high temperature gas is preferably circulated through the batch-type filter with a vacuum pump. The temperature of the gas circulated is preferably from 50 to 80°C. When the temperature of the gas circulated is lower than the crystallization temperature, the mother liquid that is contained in the recovery adduct cake is solidified, thereby making worse the filtrating property. On the other hand, when the temperature is higher than 80°C, the dissolution of the recovery adduct is promoted too much, thereby the recovery percentage of crystals is lowered.
The batch-type filter is equipped with a tray placed therein. The tray has a filter cloth stretched thereon. Above the tray having the filter cloth, is disposed a washing liquid spraying apparatus that is used to wash the filtered recovery adduct cake. The slurry liquid from the crystallizer is introduced onto the face of the filter cloth stretched on the tray so as to separate into solid and liquid. Namely, the batch-type filter is operated in accordance with the following run cycle: (i) supplying slurry, (ii) filtration, (iii) washing and dehydration of the recovery adduct cake on the filter cloth with a washing liquid, and (iv) discharging of the washed recovery adduct cake. The tray has an outlet port for drawing out a liquid phase such as filtrate or washing liquid. The washed recovery adduct cake is discharged out of the apparatus by turning over the tray. The recovery adduct cake discharged is melted in a melting vessel and then circulated to the concentration step (B) and/or crystallization and solid/liquid separation step (C).
Examples of the washing liquid for the recovery adduct cake used in the batch-type filter may include preferably phenol and phenol containing water.

The composition of the liquid phase (mother liquid) obtained after the solid/liquid separation with the batch-type filter is as: from 45 to 70% by mass of phenol, from 5 to 15% by mass of bisphenol A, and from 20 to 40% by mass of by-products such as 2,4-isomer. The reacted by-products such as 2,4-isomer are contained in a large amount, but phenol is also contained in a large amount. Therefore, phenol is recovered from the liquid phase using a packed distillation column and the like, and high boiling point compounds that are residues and contain a large amount of the by-products, colored substances or the like are discharged out of the reaction system as tar. Due to this, impurities are not accumulated in the reaction system, and high-grade bisphenol A is obtained as a product. The high boiling point compounds discharged out of the reaction system are disposed by a usual process using an incinerator or the like.
Recovery of phenol with the aforementioned packed distillation column or the like is carried out usually under a pressure inside of the column of from about 4 to 33 kPa and at a temperature inside of the column of from about 120 to 180°C. The recovery of phenol is continued until the amount of phenol remained in the residues is decreased to 20% by mass or lower and preferably from about 5 to 18% by mass. The phenol recovered in this step can be reused as a washing liquid in the crystallization and solid/liquid separation step or as a source material for the reaction, for example.

### (F) Adduct decomposition step

The adduct crystals recovered by solid/liquid separation in the above crystallization and solid/liquid separation step (C) are transformed into high-purity bisphenol A after phenol is removed in the adduct decomposition step. For example, usually the adduct crystals are decomposed into bisphenol A and phenol by heating and melting them at from 100 to 160°C. Most of the phenol is removed from resulting melt with an evaporator or the like, and then the remaining phenol is further removed by steam stripping so as to obtain a melt of bisphenol A.

### (G) Prilling step

The melt of bisphenol A obtained in the adduct decomposition step is fed to the head of a prilling tower and sprayed through a number of holes opened in a nozzle plate placed at the head of the tower. The melt thus sprayed is cooled with a circulating gas flowing upward from the bottom of the prilling tower. The resulting granular solid called as prill is taken out from the bottom of the tower to provide a product bisphenol A.

The present invention also provides an apparatus for recovering bisphenol A. The apparatus includes a crystallizer that is equipped with a jacket and has a function of scratching off an adduct of bisphenol A and phenol deposited on the inside wall thereof with a scraper blade and a batch-type filter having a washing function.
The apparatus for recovering of the present invention is effective for recovering an adduct of bisphenol A and phenol from a liquid that contains bisphenol A and has a high impurity concentration or a high viscosity, and can be suitably applied to the isomerized liquid treated in the aforementioned step (D), for example.

### [Examples]

Hereinafter, the present invention will be described in detail with reference to the following examples, but it should be construed that the present invention is in no way limited to those examples.

### Example 1

A bisphenol A solution that was a liquid treated in the isomerization step and contained 17.7% by mass of bisphenol A, 7.3% by mass of impurities such as isomer etc., and the remainder of phenol was obtained. In order to prevent impurities from being concentrated in the reaction system, a part of the solution obtained, that is 2,000 kg/hr, was fed to the recovery step. Firstly, phenol was evaporated with a evaporator to regulate the amount of the solution at 1,481 kg/hr (bisphenol A: 23.9% by mass, impurities: 9.9% by mass, and the remainder was phenol) before the solution was fed to a crystallizer, and then the solution was fed to the crystallizer (scratch-type crystallizer, manufactured by Tsukishima Kikai Co., Ltd.) having a function of scratching off with a scraper blade. The scratch-type crystallizer was operated while the temperature inside of the crystallizer was adjusted at 45°C by regulating the difference between the temperature of the cooling water in the external jacket and the temperature inside of the crystallizer at about 15°C. A slurry liquid obtained in the scratch-type crystallizer was fed to a tray filter manufactured by Tsukishima Kikai Co., Ltd. that was a batch-type filter. Filtration and washing with phenol were carried out while a 70°C temperature gas was circulated through the tray filter with a vacuum pump. The filtration time is 10 minutes and the time taken by washing, filtrating, and other operations was 5 minutes, that is, 15 minutes in all were taken by one cycle. Namely, the slurry liquid was treated at a rate of four cycles per hour.
The phenol used for washing was fed in almost the same amount of the recovery adduct. The recovery adduct cake obtained with the tray filter was melted and the yield thereof was 530 kg/hr (bisphenol A: 219 kg/hr, impurities: 16 kg/hr, and the remainder was phenol). The resulting recovery adduct cake was high-purity, having a remaining impurity concentration of 3% by mass. The recovery adduct cake was melted in a melting vessel and the resulting melt was fed to the concentration step to obtain a final product. The mother liquid discharged from the tray filter was concentrated in a distillation column until the phenol concentration become 5% by mass, and then drawn out from the bottom of the distillation column as tar in an amount of 279 kg/hr (bisphenol A: 134 kg/hr, impurities: 131 kg/hr, and phenol: 14 kg/hr).
The scratch-type crystallizer and tray filter, both were allowed to operate stably continuously over a year.

### Comparative Example 1

A solution in an amount of 1,481 kg/hr (bisphenol A: 23.9% by mass, impurities: 9.9% by mass, and the remainder was phenol) was adjusted, through the same treatment process as Example 1, so as to supply the solution to a crystallizer. After water required for heat removal was added to the adjusted solution, the solution was fed to a vacuum-vaporization crystallizer. The amount of the water added was regulated in a manner that the temperature inside of the vacuum-vaporization crystallizer was adjusted at 45°C under a pressure of 2.53 kPa-G. The resulting slurry liquid was treated with the tray filter manufactured by Tsukishima Kikai Co., Ltd. that was a batch-type filter, similarly to Example 1. The recovery adduct cake obtained in the tray filter was 425 kg/hr (bisphenol A: 176 kg/hr, impurities: 12 kg/hr, and the remainder was phenol). The recovery adduct cake was melted in a melting vessel, and the resulting melt was fed to the concentration step to obtain a final product. The mother liquid discharged from the tray filter was dehydrated in a dehydrating column, concentrated in a packed column until the phenol concentration became 5% by mass, and then drawn out from the bottom of the column as tar in an amount of 309 kg/hr (bisphenol A: 178 kg/hr, impurities: 134 kg/hr, and phenol: 16 kg/hr).
As described above, the recovery of bisphenol A in the form of crystals through the vacuum-vaporization crystallization using water was lower by about 20 percents as compared with the external cooling crystallization using phenol as a solvent. The amount discharged as tar increased by about 15%. As a result, unit of raw material became worse.

### Comparative Example 2

The same treatment as Example 1 was carried out except that a drum filter was used as a solid/liquid separator in place of the batch-type filter. The adduct cake obtained with the drum filter was 663 kg/hr (bisphenol A: 235 kg/hr, impurities: 47 kg/hr, and the remainder was phenol). The adduct cake obtained was melted in a melting vessel and the resulting melt was fed to the concentration step to obtain a final product. The mother liquid discharged from the drum filter was dehydrated in a dehydrating column, concentrated in a packed column until the phenol concentration become 5% by mass, and then drawn out from the bottom of the column as tar in an amount of 228 kg/hr (bisphenol A: 118 kg/hr, impurities: 99 kg/hr, and phenol: 11 kg/hr).
As described above, when the drum filter was used in place of the tray filter, due to the lack of time required for filtrating and washing the recovery adduct cake, the amount of impurities fed to the concentration step increases by three times as compared with the case where the tray filter was used. As a result, the impurities increased in the reaction system and eventually the product quality became worse.

### [Industrial Applicability]

In a process for recovering bisphenol A from an isomerized liquid, the present invention provides a method and an apparatus for recovering bisphenol A, wherein high-purity bisphenol A adduct is recovered and the recovered bisphenol A adduct is returned to a concentration step and/or a crystallization and solid/liquid separation step.

## Claims

1. In a process of producing bisphenol A comprising: (A) reacting an excess amount of phenol with acetone in the presence of an acidic catalyst; (B) concentrating a reaction mixture obtained in the step (A); (C) crystallizing an adduct of bisphenol A and phenol by cooling the concentrated reaction mixture obtained in the step (B) and solid/liquid separating it into the adduct and a mother liquid; (D) isomerizing all of the mother liquid obtained in the step (C) by treating the mother liquid with an isomerization catalyst; (E) recovering an adduct of bisphenol A and phenol from the liquid isomerized in the step (D); (F) decomposing the adduct of bisphenol A and phenol obtained in the step (C) by removing phenol from the adduct to obtain a bisphenol A melt; and (G) granulating the bisphenol A melt obtained in the step (F) into product prill,
a method for recovering bisphenol A in the foregoing step (E) comprising:
crystallizing an adduct of bisphenol A and phenol in the presence of phenol by supplying the liquid isomerized in the step (D) into a crystallize having an external jacket and a function of scratching off a deposit on the inside wall of the crystallizer with a scraper blade while cooling water is passed through the external jacket so as to cool the inside of the crystallizer;
scratching off an adduct deposited on the inside wall of the crystallizer;
filtering and washing the adduct slurry with a batch-type filter having a washing function and serving as a solid/liquid separator; and
circulating the resulting adduct to the step (B) and/or the step (C).

2. The method for recovering bisphenol A according to claim 1, wherein
a washing liquid used in the batch-type filter having a washing function is selected from phenol, phenol containing water, and phenol containing bisphenol A

3. An apparatus for recovering bisphenol A comprising:
a crystallizer having a jacket and a function of scratching off an adduct of bisphenol A and phenol deposited on the inside wall of the apparatus with a scraper blade; and
a batch-type filter having a washing function.
